# EUROPEAN PATENT SPECIFICATION

(11) **EP 3 399 958 B1**
(45) Date of publication and mention of the grant of the patent: **06.05.2020**
(21) Application number: 16806149.7
(22) Date of filing: 07.12.2016
(51) Int. Cl.: A61K 8/26, A61K 8/44, A61Q 15/00, A61K 8/894, A61K 8/19, A61K 8/06

(54) **ANTIPERSPIRANT COMPOSITIONS**
SCHWEISSHEMMENDE ZUSAMMENSETZUNGEN
COMPOSITIONS ANTI-TRANSPIRANTES

(30) Priority: 07.01.2016 EP 16150417
(43) Date of publication of application: 14.11.2018
(73) Proprietor: Unilever PLC, London, Greater London EC4Y 0DY (GB); Unilever NV, 3013 AL Rotterdam (NL)
(72) Inventor: ZDRAVKOVA, Aneliya, Nikolova, Bebington Wirral Merseyside CH63 3JW (GB)
(74) Representative: Whaley, Christopher
(86) International application number: PCT/EP2016/080034
(87) International publication number: WO 2017/118507

(56) References cited:
- WO-A1-2014/187684
- WO-A1-2014/187685
- US-A1- 2011 038 902

## Description

The present invention is concerned with antiperspirant compositions and with methods of making the same. It is particularly concerned with water-in-oil emulsion compositions comprising activated basic aluminium chloride (herein BAC) antiperspirant actives and their manufacture.

Certain calcium activated BAC actives are commercially available and their preparation and use are disclosed in numerous publications.

Activated BAC samples have also been prepared using water-soluble calcium acids, particularly with a further adjunct such as an amino acid, hydroxyl acid, or betaine. Some of these samples could be formulated into aqueous compositions without the antiperspirant losing all of its enhanced activity.

EP 1,104,282 (Gillette) discloses a means of producing activated BAC samples using a water soluble calcium salt and an amino acid or a hydroxy acid.

US 6,911,195 (Gillette) discloses water-in-oil emulsion gels comprising aluminium-zirconium antiperspirant salts activated using calcium ions.

US 5,955,065 (Gillette) discloses anhydrous suspension formulations comprising particulate BAC and aluminium-zirconium antiperspirant salts activated using calcium ions.

US 6,942,850 (Gillette) discloses aqueous alcoholic composition comprising aluminium-zirconium antiperspirant salts activated using calcium ions.

WO 2009/044381 (P&G) discloses water-in-oil emulsion sticks comprising BAC and aluminium-zirconium antiperspirant salts activated using calcium ions.

US 7,704,531 (Colgate) discloses compositions comprising an active system made from combining an aluminium or aluminium-zirconium salt, a calcium salt, and a betaine.

US 2011/038823 and US 2011/038902 (Dial/Henkel) discloses water-in-oil emulsion sticks comprising an antiperspirant active prepared by combining BAC, calcium chloride and glycine.

US 2007/196303, US 2007/0020211, WO 2008/063188, US 2008/0131354 and US 7,087,220 (Summit and Reheis) each describe methods of making calcium-activated antiperspirant salts.

WO 2014/187685 and WO 2014/187802 (Unilever) disclose compositions comprising calcium and glycine activated aluminium sesquichlorohydrate and methods of making the same.

The present invention is particularly concerned with BAC compositions comprising aluminium sesquichlorohydrate (herein ASCH) of chemical formula Al₂OH_{4.4}Cl_{1.6} to Al₂OH_{4.9}Cl_{1.1}. This material is commercially available, but its formulation and use described herein are new and deliver unexpected benefits.

In a first aspect of the present invention, there is provided a water-in-oil emulsion antiperspirant composition according to claim 1.

In a second aspect of the present invention, there is provided a method of manufacture of an antiperspirant composition according to the first aspect of the invention.

In a third aspect of the present invention, there is provided a method of attaining an antiperspirant benefit comprising the topical application to the surface of the human body of a composition according to the first aspect of the invention, especially when manufactured in accordance with the second aspect of the invention.

The compositions of the present invention are cosmetic compositions and their use to treat perspiration is a cosmetic use as opposed to a therapeutic one.

Herein, the "activation mixture" refers to the mixture of basic aluminium chloride salt of formula Al₂OH_{4.4}O_{1.6} to Al₂OH_{4.9}Cl_{1.1}, water-soluble calcium salt, amino acid, and water.

The choice of BAC salt used is important to the success of the present invention. We have found that surprisingly good results are found on using BAC salts commonly referred to as aluminium sesquichlorohydrate (herein ASCH) having the chemical formula Al₂OH_{4.4}Cl_{1.6} to Al₂OH_{4.9}Cl_{1.1}. Most commercial ASCH samples are of chemical formula Al₂OH_{4.7}Cl_{1.3} to Al₂OH_{4.9}Cl_{1.1} and it is preferred to use BAC salts of this formula.

The surprisingly good results referred to in the above paragraph include surprisingly good antiperspirancy performance. In addition, compositions prepared according to the present invention have remarkable storage stability, maintaining their good performance for many months.

The enhancement of the antiperspirancy performance observed with the use of activated BACs as described herein in water-in-oil emulsion antiperspirant compositions is surprisingly high. Water-in-oil emulsion antiperspirant compositions are notoriously difficult to obtain good antiperspirancy results with and the present invention addresses that problem.

The BAC salt used in the present invention has aluminium to chloride molar ratio of from 1.25:1 to 1.82:1 and preferably 1.54:1 to 1.82:1.

In order for the antiperspirant to become activated, it is important to have sufficient calcium present relative to the amount of aluminium present. The molar ratio of calcium to aluminium is typically at least 1:40, preferably at least 1:30 and more preferably at least 1:20. It is not advantageous to have the calcium concentration in excess of the aluminium concentration, indeed it is preferred that the calcium concentration is no more than half that of the aluminium concentration and more preferred that it is no more than a fifth of said concentration. For the preferred molar ratios of calcium to aluminium of at least 1:40 and at least 1:20, it is independently preferred that this ratio is no greater than 1:2 and more preferred that it is no greater than 1:5.

In particularly preferred embodiments, the molar ratio of calcium to aluminium is at least 1:15 and preferably no greater than 1:5 and in especially preferred embodiments it is at least 1:10 and preferably no greater than 1:5.

Herein, references to molar amounts and ratios of "aluminium" are calculated on the basis of mono-nuclear aluminium, but include aluminium present in poly-nuclear species; indeed, most of the aluminium in the salts of relevance is present in poly-nuclear species.

In order for the antiperspirant to become activated, it is important to have sufficient amino acid present relative to the amount of aluminium present. The molar ratio of amino acid to aluminium is preferably at least 1:20, more preferably at least 1:10 and most preferably at least 1:5. It is not advantageous to have the amino acid concentration in excess of the aluminium concentration; hence, the molar amino acid to aluminium is preferably from 1:20 to 1:1, more preferably from 1:10 to 1:1 and most preferably from 1:5 to 1:1.

In particularly preferred embodiments, the molar ratio of amino acid to aluminium is at least 1:4 and preferably no greater than 1:1 and in especially preferred embodiments it is at least 1:3 and preferably no greater than 1:1.

The presence of both calcium and amino acid is essential for the success of the present invention. In preferred embodiments, the molar ratio of calcium to aluminium is at least 1:40 and the molar ratio of amino acid to aluminium is at least 1:20. In further preferred embodiments the molar ratio of calcium to aluminium is at least 1:20 and the molar ratio of amino acid to aluminium is at least 1:10. In particularly preferred embodiments the molar ratio of calcium to aluminium is from 1:20 to 1:5 and the molar ratio of amino acid to aluminium is from 1:10 to 1:1.

The above indicated preferences for calcium to aluminium molar ratio and/or amino acid to aluminium molar ratio lead to compositions of higher Band III content (*vide infra*) and, in general, higher antiperspirancy performance. It will be noted that higher Band III content is generally indicative of higher antiperspirancy performance.

The amino acid for use in the present invention is glycine.

The activation process generally produces a mixture of aluminium species having a relatively high content of what is commonly termed Band III material, as determined by SEC (Size Exclusion Chromatography) analysis. The SEC technique employed is well known in the art and is described in further detail in US 4,359,456 (Gosling). The SEC band commonly referred to as Band III is designated as "Peak 4" in EP 1,104,282 B1 by Gillette.

Herein, "Band III content" refers to the integrated area in the Band III region of the SEC chromatograph relative to the total integrated area in all of the regions corresponding to aluminium species; that is to say, Bands I, II, III, and IV.

In particular embodiments of the invention, compositions according to the invention intended for use as antiperspirant compositions preferably have a Band III content of at least 30% and more preferably at least 50%.

In the heat activation of the BAC salt as described herein, it is preferred that the activation mixture is heated for sufficient time for the Band III content of the aluminium species to become at least 30% and more preferably at least at least 50%.

In the heat activation of the BAC salt as described herein, the activation mixture is heated to at least 65°C, preferably to at least 75°C, and more preferably to at least 85°C. The heat activation is typically performed for at least one hour.

Herein, "free water" excludes any water of hydration associated with the antiperspirant salt or other component added to a particular composition, but includes all other water present.

Herein, amounts and concentrations of ingredients are percentages by weight of the total composition, unless otherwise indicated and ratios are ratios by weight.

Herein, the terms "oil" and signifies a water-insoluble organic material that is liquid at 20°C. Any material having a solubility of less than 0.1g/100g at 20°C is considered insoluble.

The water-in-oil emulsion compositions of the present invention preferably have a balance of aqueous phase to oil phase that is greater than 1:1, i.e. it is preferred that the internal phase is of greater total weight than the continuous phase. The ratio of aqueous phase to oil phase is particularly preferably from greater than 50:50 to 82:18, especially from 70:30 to 82:18 and most preferably from 72:28 to 80:20.

An essential component of the invention is the oil continuous phase in which the aqueous phase is suspended. It is preferred that the oil continuous phase comprises a silicone oil and particularly preferred that the silicone oil or oils comprise the majority, i.e. greater than 50% by weight, of the oils present in the composition. It is especially preferred that the silicone oil comprises greater than 90% by weight of the oils present in the composition.

Preferred silicone oils are linear and cyclic dimethicone oils. Particularly preferred are cyclic dimethicones, such as cyclopentasiloxane for example. Other silicone oils that can be incorporated are phenyl or diphenylene substituted dimethicones, for example Dow Corning 200 350cps or Dow Corning 556.

The water-in-oil antiperspirant compositions of the present invention comprise a non-ionic emulsifier system comprising silicone emulsifiers. Such an emulsifier system conveniently has a mean HLB value in the region of from about 3 to about 8 and particularly from about 4 to about 6. Such a mean HLB value is preferably obtained by selecting two different silicone emulsifiers, one having a higher HLB and one having a lower HLB. It is particularly preferred that the two emulsifiers differ in HLB by no more than 2 units. Such preferred emulsifier systems can lead to both high antiperspirancy performance and superior storage stability, particularly at high temperatures.

Herein, mean HLB values are calculated as weighted means.

The silicone emulsifiers are ethoxylated and/or propoxylated silicone ethers. The emulsifier system comprises two different emulsifiers, one hasing an alkyl terminus and the other not having an alkyl terminus. Such emulsifier systems have been found to give both high antiperspirancy performance and superior storage stability, particularly at high temperatures.

The total proportion of emulsifiers in the composition is usually at least 1 % and particularly at least 1.5% by weight. Commonly, the emulsifiers are not present at above 10%, often not more than 7% by weight and in many preferred embodiments up to 6% by weight. An especially desirable concentration range for the emulsifiers is from 1.5 to 5% by weight.

An additional optional component for use in accordance with the present invention is a fragrance oil, sometimes alternatively called a perfume oil. The fragrance oil may comprise a single fragrance or component more commonly a plurality of fragrance components. Herein, fragrance oils impart an odour, preferably a pleasant odour, to the composition. Preferably, the fragrance oil imparts a pleasant odour to the surface of the human body the composition is applied to the same.

The amount of fragrance oil in the composition is commonly up to 3% advantageously is at least 0.5% and particularly from 0.8% to 2%.

In certain embodiments, it is preferred to include an oil, other than a fragrance oil or silicone oil that has a relatively low viscosity, by which is meant less 250 cS (mm².s⁻¹). Such oils can improve the sensory properties of the composition on application and can lead to other benefits such as emolliency.

Suitable oils can be selected from alkyl ether oils having a boiling point of above 100°C and especially above 150°C, including polyalkyleneglycol alkyl ethers. Such ethers desirably comprise between 10 and 20 ethylene glycol or propylene glycol units and the alkyl group commonly contains from 4 to 20 carbon atoms. The preferred ether oils include polypropylene glycol alkyl ethers such as PPG-14-butylether and PPG-15-stearyl ether.

Suitable oils can include one or more triglyceride oils. The triglyceride oils commonly comprise the alkyl residues of aliphatic C₇ to C₂₀ alcohols, the total number of carbon atoms being selected in conjunction with the extent of olefinic unsaturation and/or branching to enable the triglyceride to be liquid at 20°C. One example is jojoba oil. Particularly preferably, in the triglyceride oil the alkyl residues are linear C₁₈ groups having one, two or three olefinic degrees of unsaturation, two or three being optionally conjugated, many of which are extractable from plants (or their synthetic analogues), including triglycerides of oleic acid, linoleic acid, conjugated linoleic acids, linolenic acid, petroselenic acid, ricinoleic acid, linolenelaidic acid, trans 7-octadecenoic acid, parinaric acid, pinolenic acid, punicic acid, petroselenic acid and stearidonic acid. Suitable oils can include those derived from unsaturated C₁₈ acids, including coriander seed oil, impatiens balsimina seed oil, parinarium laurinarium kernel fat oil, sabastiana brasilinensis seed oil, dehydrated castor seed oil, borage seed oil, evening primrose oil, aquilegia vulgaris oil, sunflower (seed) oil and safflower oil. Other suitable oils are obtainable from hemp, and maize corn oil. An especially preferred oil is sunflower (seed) oil.

Further suitable oils, that can also be emollient oils, comprise alkyl or alkyl-aryl ester oils having a boiling point of above 150°C (and a melting point of below 20°C). Such ester oils include oils containing one or two alkyl groups of 12 to 24 carbon atoms length, including isopropyl myristate, isopropyl palmitate and myristyl palmitate. Other non-volatile ester oils include alkyl or aryl benzoates such C₁₂₋₁₅ alkyl benzoate, for example Finsolv TN™ or Finsolv Sun™.

Other components that may be present include short chain (C₂-C₄) alcohols and especially polyols such glycerol, ethylene glycol, propylene glycol and polymers thereof, in particular poly(ethylene glycol) and poly(propylene glycol). Poly(ethylene glycol) of average molecular weight 200 to 600 is a preferred component. Such components may add to the sensory properties of the composition and, when included, are typically present at from 0.5 to 10% of the total composition.

The compositions of the present invention are suitable for dispensing via a roll-on dispenser, i.e. they are capable of flow. Suitable roll-on dispensers for use with compositions of the present invention include upright dispensers, such as described in EP1175165, or inverted dispensers, such as described in US6511243 or WO05/007377. In using such dispensers, the composition is applied by rolling the ball of the dispenser across the skin surface, depositing a film of fluid on the skin.

The viscosity of compositions according to the invention is preferably 10,000 mPa.s or less, more preferably 8,000 mPa.s or less and most preferably 7,000 mPa.s or less, such viscosities enhancing the flow properties of the compositions.

It has also been found desirable to maintain a certain minimum viscosity as this can enhance stability, particularly at high temperatures, such as 50°C. Thus, it is preferred that the composition has a preferred viscosity of at least 1,000 mPa.s, a more preferred viscosity of at least 2,000 mPa.s and a most preferred viscosity of at least 4,000 mPa.s.

In order to achieve both good flow properties and good stability, compositions according to the invention have a viscosity that is preferably from 1,000 to 10,000 mPa.s, more preferably from 2,000 to 8,000 mPa.s and most preferably from 4,000 mPa.s to 7,000 mPa.s.

Herein, indicated viscosities are measured at 10 rpm at ambient temperature.

In some embodiments, the water-in-oil antiperspirant emulsions as described herein may be mixed with a volatile propellant and used as an aerosol product, dispensed from a conventional aerosol dispenser. Such antiperspirant aerosol compositions comprise a water-in-oil emulsion antiperspirant composition as described in the first aspect of the invention and a volatile propellant.

Herein, a volatile propellant is one having a boiling point of below 10°C and especially those with a boiling point below 0°C. Such materials are typically in liquefied form at the pressure within the container from which they are dispensed.

Suitable volatile propellants include trichlorofluoromethane, trichlorotrifluoroethane, difluoroethane, propane, butane, isobutane, or combinations thereof. It is especially preferred to employ liquefied hydrocarbon gases, and especially C₃ to C₆ hydrocarbons, including propane, butane, isobutane, pentane and isopentane and mixtures of two or more thereof. Of these especially preferred propellants, isobutane, isobutane/propane, butane/propane and mixtures of propane, isobutane and butane are most preferred. The amount used is typically from 5 to 95% and preferably from 30 to 90% by weight of the total composition.

A typical method of manufacture of compositions according to the present invention comprises the steps of heating to at least 65°C an aqueous solution of basic aluminium chloride salt is of formula Al₂OH_{4.4}Cl_{1.6} to Al₂OH_{4.9}Cl_{1.1} with water-soluble calcium salt and amino acid, with the molar ratio of calcium to aluminium being at least 1:20 and the molar ratio of amino acid to aluminium being at least 1:10, and emulsifying the resulting solution into an oily continuous phase.

The first stage of the method of manufacture as described in the above paragraph causes the basic aluminium chloride salt to be "heat activated" by the water-soluble calcium salt and amino acid. The resulting heat activated solution is typically cooled prior to emulsification in the oily continuous phase and preferably it is also diluted with further water before or during the emulsification.

The preferences described above with regard to compositions according to the invention apply where appropriate to the typical method of manufacture of such compositions as described in the two paragraphs immediately above.

The method of attaining an antiperspirant benefit described as the third aspect of the invention (*vide supra*) may involve direct or indirect topical application to the composition surface of the human body. In each of the methods described in this paragraph, the composition is preferably applied to the underarm regions of the human body.

### Examples

In the following examples, all percentages, parts and ratios are by weight, unless otherwise indicated.

The Chlorohydrol 50 solution was an aqueous solution comprising approximately 50% by weight of hydrated aluminium chlorohydrate (ACH), equating to approximately 40% anhydrous ACH, and was obtained from SummitReheis. We measured its Al content at 12.9% by weight. The ACH has an approximate general formula Al₂(OH)₅Cl and an Al:Cl ratio of approximately 2:1.

The Reach 301L solution was approximately 40% ASCH (based on anhydrous solids) and was obtained from Summit-Reheis. The ASCH had an approximate general formula of Al₂(OH)_{4.8}Cl_{1.2} and an Al:Cl ratio of approximately 1.67:1.

The calcium chloride dihydrate and glycine were both ex Sigma-Aldrich.

The cyclopentasiloxane was PMX-0245 ex Xiameter.

The glycerine was Pricerine 9091 ex Croda.

The "SSO" was sunflower seed oil or *Helianthis Annuus* seed oil ex Aarhus Karlshamn.

The Abil EM97 was Bis-PEG/PPG-14/14 Dimethicone and cyclopentasiloxane, 85:15, ex Evonik.

The Abil EM90 was Cetyl PEG/PPG-10/1 Dimethicone ex Evonik.

Two heat activated BAC salt solutions were prepared as followings.
51.07 parts of water, 40.00 parts of Reach 301L solution, 2.65 parts of calcium chloride dihydrate and 6.27 parts of glycine were weighed into a container and the solution was stirred until it was clear. The resulting solution was heated at 85°C for 18 hours in a capped glass vessel and was then quickly cooled back to approximately 35°C and then allowed to cool naturally back to room temperature to give Solution 1.

Solution 2 was prepared in exactly the same way as Solution 1, except that the solution was heated at 85°C for 5 hours.

Solutions 1 and 2 were used in the preparation of the antiperspirant roll-on compositions indicated in Table 1. The ASCH, CaCl₂ and glycine came solely from the heat activated BAC solution and the amounts indicated are those present in the final composition. The BAC solution used in the preparation of Comparative Example A was Chlorohydrol 50 solution (40% anhydrous ACH).

The roll-on compositions indicated in Table 1 were manufactured at 1 L scale using following process.

In a first beaker, the Abil EM90, Abil EM97, and cyclopentasiloxane were stirred until homogenous. In a second beaker, the glycerol, BAC salt solution and any additional water were stirred until homogenous.

The aqueous phase was slowly added to the oil phase with constant stirring using a Heidolph stirrer and increasing rpm from 200 to 450, stirring being maintained at 450 rpm for 5 minutes after completion of the addition of the aqueous phase.

Using a Silverston homogeniser, the emulsion was sheared at 5000 rpm for 10 seconds bursts until the viscosity reached 10,000 to 12,000 mPa.s (at 10 rpm).

The emulsion was returned to the Heidolph and stirred at 300 rpm during the addition of the sunflower seed oil and then the perfume to give the final product, which was loaded into conventional roll-on applicators.

**Table 1: Antiperspirant Roll-On Composition**

| Example: | 1 | 2 | 3 | 4 | A |
|---|---|---|---|---|---|
| | % w/w | | | | |
| Solution 1 | 50.00 | -- | -- | -- | -- |
| Solution 2 | -- | 50.00 | 69.00 | 69.00 | -- |
| ASCH | 8 | 8 | 11 | 11 | -- |
| CaCl₂ | 1.33 | 1.33 | 1.82 | 1.82 | -- |
| Glycine | 3.13 | 3.13 | 4.31 | 4.31 | -- |
| ACH solution | -- | -- | -- | -- | 30 |
| Cyclopentasiloxane | 20 | 20 | 20 | 20 | 20 |
| Glycerine | 4 | 4 | 4 | 4 | 4 |
| SSO | 4 | 4 | 0.5 | 4 | 4 |
| Abil EM97 | 2 | 1 | 1 | 1 | 1.75 |
| Abil EM90 | -- | 1 | 1 | 1 | 1.75 |
| Perfume | 1 | 1 | 1 | 1 | 1 |
| Water | To 100 | To 100 | To 100 | -- | To 100 |
| Viscosity | 4020 | 5930 | 6170 | 6050 | 3500 |

Viscosities (in mPa.s) of the final products are also indicated in Table 1. All viscosities were measured with a Brookfield rheometer (spindle RV4) at 10 rpm.

"Sweat weight reduction" (SWR) results were obtained on some of the compositions using a test panel of 30 female volunteers. Test operators applied a test composition (0.30g) to one axilla and 0.30g of non-antiperspirant deodorant body spray to the other axilla of each panellist. This was done once each day for three days. After the third application, panellists were requested not to wash under their arms for the following 24 hours.

24 hours after the third and final product application, the panellists were induced to sweat in a hot-room at 40°C (±2°C) and 40% (±5%) relative humidity, for 40 minutes. After this period, the panellists left the hot-room and their axillae were carefully wiped dry. Pre-weighed cotton pads were then applied to each axilla of each panellist and the panellists re-entered the hot-room for a further 20 minutes. Following this period, the pads were removed and re-weighed, enabling the weight of sweat generated to be calculated.

The sweat weight reduction (SWR) for each panellist was calculated as a percentage (% SWR) and the mean % SWR was calculated according to the method described by Murphy and Levine in "Analysis of Antiperspirant Efficacy Results", J. Soc. Cosmetic Chemists, 1991(May), 42, 167-197.

The SWR results obtained for Examples 1 and 2 were 42% and 33% respectively. The SWR result for Comparative Example A was much poorer, only 8%, despite this composition containing a higher percentage of antiperspirant active (12%, based on anhydrous ACH) than the examples according to the invention.

High temperature stability trials were also carried out on the compositions detailed in Table 1. Samples were stored in sealed vessels at 50°C and their appearance monitored.

Example 1, despite giving good SWR and good stability at ambient temperature, was not very stable at 50°C and started to phase separate after 8 hours. Example 2, having the dual emulsifier system of Abil EM97 and Abil EM90 was stable for up to 21 days at 50°C.
Examples 3 and 4, both having Abil EM97 and Abil EM90 present, both exhibited good stability at 50°C. Example 3 was stable for 56 days and Example 4 was stable for 21 days, the improved stability of the former being attributable to the high content of silicone oil present compared with other oils.

The moisturising qualities of Examples 1, 3 and 4 were also tested. Each Example was found to give skin hydration scores significantly greater than those for the market-leading moisturising antiperspirant roll-on.

## Claims

1. A water-in-oil emulsion antiperspirant composition comprising basic aluminium chloride salt, calcium chloride, and glycine, **characterised in that** the basic aluminium chloride salt is of formula Al₂OH_{4.4}Cl_{1.6} to Al₂OH_{4.9}Cl_{1.1} and is activated by heating this salt with calcium chloride and glycine, the molar ratio of calcium to aluminium being at least 1:20 and the molar ratio of glycine to aluminium being at least 1:10 in both the heat activation of the basic aluminium salt and in the final composition, wherein the composition comprises a non-ionic emulsifier system comprising two different ethoxylated and/or propoxylated silicone ethers, one having an alkyl terminus and the other not having an alkyl terminus.

2. A composition according to claim 1 wherein the basic aluminium chloride salt is of formula Al₂OH_{4.7}Cl_{1.3} to Al₂OH_{4.9}Cl_{1.1}.

3. A composition according to any of the preceding claims, which is capable of flow and suitable for dispensing via a roll-on dispenser.

4. A composition according to any of the preceding claims, wherein silicone oil or oils comprise greater than 50% by weight of the oils present in the oil continuous phase.

5. A method of manufacture of a water-in-oil emulsion antiperspirant composition comprising the steps of heating to at least 65°C an aqueous solution of basic aluminium chloride salt of formula Al₂OH_{4.4}Cl_{1.6} to Al₂OH_{4.9}Cl_{1.1} calcium chloride and glycine, with the molar ratio of calcium to aluminium being at least 1:20 and the molar ratio of glycine to aluminium being at least 1:10, and emulsifying the resulting solution into an oily continuous phase by use of a non-ionic emulsifier system comprising two different ethoxylated and/or propoxylated silicone ethers, one having an alkyl terminus and the other not having an alkyl terminus.

6. A method according to claim 5, wherein the heat activated solution is cooled prior to emulsification in the oily continuous phase and is also diluted with further water before or during the emulsification.

7. A method of attaining an antiperspirant benefit comprising the topical application to the surface of the human body of a composition according to any of claims 1 to 4.

## Patentansprüche

1. Schweißhemmende Wasser-in-ÖI-Emulsions-Zusammensetzung, umfassend basisches Aluminiumchloridsalz, Calciumchlorid und Glycin, **dadurch gekennzeichnet, dass** das basische Aluminiumchloridsalz die Formel Al₂OH_{4.4}-Cl_{1.6} bis Al₂OH_{4.9}Cl_{1.1} aufweist und durch Erhitzen dieses Salzes mit Calciumchlorid und Glycin aktiviert ist, wobei das molare Verhältnis von Calcium zu Aluminium mindestens 1:20 und das molare Verhältnis von Glycin zu Aluminium mindestens 1:10 sowohl bei der Hitzeaktivierung des basischen Aluminiumsalzes als auch in der endgültigen Zusammensetzung beträgt, wobei die Zusammensetzung ein nicht-ionisches Emulgatorsystem umfasst, umfassend zwei verschiedene ethoxylierte und/oder propoxylierte Siliconether, wobei einer eine Alkyl-Begrenzung und der andere keine Alkyl-Begrenzung aufweist.

2. Zusammensetzung nach Anspruch 1, wobei das basische Aluminiumchloridsalz die Formel Al₂OH_{4.7}Cl_{1.3} bis Al₂OH_{4.9}Cl_{1.1} aufweist.

3. Zusammensetzung nach einem der vorhergehenden Ansprüche, die fließfähig und zum Abgeben über einen Roll-on-Spender geeignet ist.

4. Zusammensetzung nach irgendeinem der vorhergehenden Ansprüche, wobei das Siliconöl oder die -öle mehr als 50 Gewichts-% der Öle, die in der kontinuierlichen Ölphase vorliegen, umfassen.

5. Verfahren zur Herstellung einer schweißhemmenden Wasser-in-ÖI-Emulsions-Zusammensetzung, umfassend die Schritte des Erhitzens einer wässrigen Lösung von basischem Aluminiumchloridsalz der Formel Al₂OH_{4.4}Cl_{1.6} bis Al₂OH_{4.9}Cl_{1.1}, Calciumchlorid und Glycin auf mindestens 65°C, wobei das Molverhältnis von Calcium zu Aluminium mindestens 1:20 und das Molverhältnis von Glycin zu Aluminium mindestens 1:10 beträgt, und Emulgieren der erhaltenen Lösung in einer öligen kontinuierlichen Phase unter Verwendung eines nicht-ionischen Emulgatorsystems, umfassend zwei verschiedene ethoxylierte und/oder propoxylierte Siliconether, wobei einer eine Alkyl-Begrenzung und der andere keine Alkyl-Begrenzung aufweist.

6. Verfahren nach Anspruch 5, wobei die hitzeaktivierte Lösung vor der Emulgierung in der öligen kontinuierlichen Phase gekühlt wird und auch mit weiterem Wasser vor oder während der Emulgierung verdünnt wird.

7. Verfahren zum Erlangen eines schweißhemmenden Nutzens, umfassend das topische Auftragen einer Zusammensetzung nach einem der Ansprüche 1 bis 4 auf die Oberfläche des menschlichen Körpers.

## Revendications

1. Composition d'antiperspirant en émulsion eau-dans-huile comprenant du sel de chlorure d'aluminium basique, chlorure de calcium, et de la glycine, **caractérisée en ce que** le sel de chlorure d'aluminium basique est de formule Al₂OH_{4,4}Cl_{1,6} à Al₂OH_{4,9}Cl_{1,1} et est activé en chauffant ce sel avec du chlorure de calcium et de la glycine, le ratio molaire de calcium à aluminium étant d'au moins 1:20 et le ratio molaire de glycine à aluminium étant d'au moins 1:10 à la fois dans l'activation thermique du sel d'aluminium basique et dans la composition finale, dans laquelle la composition comprend un système d'émulsionnant non-ionique comprenant deux silicone éthers éthoxylés et/ou propoxylés différents, un présentant une terminaison alkyle et l'autre ne présentant pas de terminaison alkyle.

2. Composition selon la revendication 1, dans laquelle le sel de chlorure d'aluminium basique est de formule Al₂OH_{4,7}Cl_{1,3} à Al₂OH_{4,9}Cl_{1,1}.

3. Composition selon l'une quelconque des revendications précédentes, qui est apte à l'écoulement et appropriée pour une distribution via un distributeur à bille.

4. Composition selon l'une quelconque des revendications précédentes, dans laquelle l'huile ou les huiles de silicone comprennent plus de 50 % en masse des huiles présentes dans la phase continue d'huile.

5. Procédé de fabrication d'une composition d'antiperspirant en émulsion eau-dans-huile comprenant les étapes de chauffage à au moins 65°C d'une solution aqueuse de sel de chlorure d'aluminium basique de formule Al₂OH_{4,4}Cl_{1,6} à Al₂OH_{4,9}CL_{1,1}, chlorure de calcium et glycine, avec le ratio molaire de calcium à aluminium étant d'au moins 1:20 et le ratio molaire de glycine à aluminium étant d'au moins 1:10, et émulsification de la solution résultante dans une phase continue huileuse par l'utilisation d'un système d'émulsionnant non-ionique comprenant deux silicone éthers éthoxylés et/ou propoxylés différents, un présentant une terminaison alkyle et l'autre ne présentant pas de terminaison alkyle.

6. Procédé selon la revendication 5, dans lequel la solution thermiquement activée est refroidie avant l'émulsification dans la phase continue huileuse et est également diluée avec plus d'eau avant et après l'émulsification.

7. Procédé pour atteindre un bénéfice antiperspirant comprenant l'application topique à la surface du corps humain d'une composition selon l'une quelconque des revendications 1 à 4.
